# EUROPEAN PATENT APPLICATION

(11) **EP 2 394 642 A1**
(43) Date of publication of application: **14.12.2011**
(21) Application number: 10165494.5
(22) Date of filing: 10.06.2010
(51) Int. Cl.: A61K 9/16, A61K 9/51, A61K 31/197

(54) **5-ALA ester formulations and use thereof**

(71) Applicant: UNIVERSITE DE GENEVE, 1211 Genève 4 (CH)
(72) Inventor: Lange, Norbert, 1260, Nyon (CH); Heuck, Gesine, 1227, Les Acacias Genève (CH)
(74) Representative: reuteler & cie SA

(57) **Abstract**

The present invention is directed to particles comprising 5-ALA esters salts, formulation thereof, related methods of preparation and methods of use thereof. In particular, the invention relates to particles comprising 5-ALA esters salts, formulation thereof useful in the treatment of a cancer and/or the diagnosis of a cancer cell such as in photodynamic therapy or photodynamic diagnosis.

## Description

### Field of the invention

The present invention is directed to 5-Aminolevulinic acid (5-ALA) ester mediated photodynamic therapy (PDT) and fluorescence photodetection (FD) of neoplastic diseases.

### Background of the invention

Photochemotherapy or photodynamic therapy is a technique based on the discovery that cancer cells treated with certain chemicals will die when exposed to light. This technique consists in the administering of a tumor-localizing photosensitizer (photochemotherapeutic) followed by the irradiation of the targeted tumor site with photoactivating light of a suitable wavelength, in order to activate the photosensitizing agent that in turn converts oxygen into cytotoxic forms, whereby the affected cells are killed or their proliferative potential diminished.

5-ALA is known to specifically induce the *in-situ* formation of high amounts of photoactive porphyrins in neoplastic and (non-) neoplastic cells with high cellular turnover. Beside the use of 5-ALA for treatment by PDT the use of its ability to induce preferential synthesis of photoactive porphyrins in neoplasia was suggested for improving detection of neoplastic lesions in order to guide biopsies, improve surgery, or allow for appropriate patient's management. However, the clinical development of 5-ALA-mediated phototherapy and fluorescence photodetection (FD) of neoplastic diseases, which are those of the most selective cancer treatment techniques, have been hampered by the limited local bioavailability of 5-ALA. In fact, the short plasma half-life of 5-ALA and its low bioavailability largely hinders its application for neoplastic lesions that are not accessible by topical administration.

The development of 5-ALA derivatives and especially 5-ALA esters (*WO 96*/*28412, WO 02*/*10120,* Fotinos et al., 2006, Photochemistry and Photobiology, 82: 994-1015*)* such as methylaminolevulinate (MAL) and hexylaminolevulinate (HAL), led to market approval of those for the treatment of actinic keratinosis and difficult-to-treat basal cell carcinoma (Metvix®; Galderma, Switzerland) and for improving detection of superficial bladder cancer (Hexvix®; Photocure ASA, Oslo, Norway), respectively. Salts of 5-ALA and 5-ALA derivatives (WO 2005/092838) have been further developed for improving their physicochemical properties such as for increasing their stability (e.g. lower hygroscopicity).

However, the use of 5-ALA esters remains restricted to organs that are accessible by topical administration as they have been shown to be unstable in the gastrointestinal tract when given orally or in the blood plasma. Their systemic use has shown to induce acute toxicity after bolus injections in numerous *in vivo* models (Perotti et al., 2002, British Journal of Cancer, 87: 790-795*;* Di Venosa et al., 2006, Cancer Chemotherapy and Phamracology, 58, 478-486 or Fotinos et al., 2006, Photochemistry Photobiology, 82: 994-1015). Furthermore, the systemic use of 5-ALA esters induces the formation of porphyrins in a much less effective way than 5-ALA.

Therefore, important life-threatening diseases including brain cancer, prostate cancer, mama carcinoma, breast cancer, ovarian cancer, lung cancer, and lymphoma can hardly be detected/treated by 5-ALA-mediated phototherapy technology. Thus, there is a big need for alternative photochemotherapeutic agents or agents for fluorescence photodetection via parenteral routes, namely oral, intravenous, intraperitonal, intramuscular, or subcutaneous.

### Summary of the invention

The invention relates to the unexpected finding of salts of 5-ALA esters according to the invention which are particularly suitable for their efficient encapsulation into biocompatible polymeric matrixes forming particles such as micro- or nanoparticles. The invention is further related to the unexpected finding of formulations of 5-ALA esters according to the invention presenting a controlled release of 5-ALA esters once systemically administrated, an increased bioavailability and increased plasma half-life together with a reduced toxicity. The invention further relates to particles according to the invention comprising 5-ALA esters salts, formulations thereof, related methods of preparation and methods of use thereof, those particles presenting a substantially increased ability in inducing the formation of photoactive porphyrins in cancer cells as compared to the corresponding free salt. In particular, the methods, uses, formulations and compositions according to the invention are useful in patients in the diagnosis of a cancer cell and/or treatment of a cancer.

As a consequence, formulations according to the invention advantageously present an increased therapeutic index as compared to known agents useful for photochemotherapy and fluorescence photodetection of neoplastic diseases.

A first aspect of the invention provides a particle for systemic administration in a human or animal host, said particle comprising a biocompatible polymer and a salt of a 5-aminolevulinic acid ester, wherein the salt is selected from an aryl sulfonate, an aryl phosphate and an aryl nitrate salt or mixtures thereof.

A second aspect of the invention provides a particle according to the invention for use as a medicament.

A third aspect of the invention provides a pharmaceutical formulation comprising a particle according to the invention and at least one pharmaceutically acceptable carrier.

A fourth aspect of the invention provides a kit comprising in one or more container(s) a formulation according to the invention together with instruction of use of said formulation. A fifth aspect of the invention provides a formulation according the invention for use as a medicament.

A sixth aspect of the invention provides a particle or a formulation according the invention for the treatment or repression of a disease or disorder including any malignant, pre-malignant and non-malignant abnormalities responsive to photochemotherapy, including, but not limited to, tumors or other hyperproliferative conditions such as cancers, skin disorders such as psoriasis, skin cancer, or actinic keratosis, infectious diseases (e.g. viral, bacterial, fungal infections), inflammatory diseases like Morbus Crohn, arthritis and rheumatoid arthritis, Barrett's esophagus or arterial restenosis.

A seventh aspect of the invention provides a particle or a formulation according the invention for the treatment or repression of a cancer or for the detection of a cancer cell.

An eighth aspect of the invention provides a use of a particle according to the invention or a formulation thereof for the preparation of a pharmaceutical composition for the treatment or repression of a disease or disorder including any malignant, pre-malignant and non-malignant abnormalities responsive to photochemotherapy, including, but not limited to, tumors or other hyperproliferative conditions such as cancers, skin disorders such as psoriasis, skin cancer, or actinic keratosis, infectious diseases (e.g. viral, bacterial, fungal infections), inflammatory diseases like Morbus Crohn, arthritis and rheumatoid arthritis, Barrett's oesophagus or arterial restenosis.

A ninth aspect of the invention provides a use of a particle according to the invention or a formulation thereof for the preparation of a pharmaceutical composition for the treatment or repression of a cancer or for detection of a cancer cell.

A tenth aspect of the invention provides a process for the preparation of a particle according to the invention.

An eleventh aspect of the invention provides a method of treating or repressing a disease or disorder including any malignant, pre-malignant and non-malignant abnormalities responsive to photochemotherapy, including, but not limited to, tumors or other hyperproliferative conditions such as cancers, skin disorders such as psoriasis, skin cancer, or actinic keratosis, infectious diseases (e.g. viral, bacterial, fungal infections), inflammatory diseases like Morbus Crohn, arthritis and rheumatoid arthritis, Barrett's oesophagus or arterial restenosis, said method comprising administering in a subject in need thereof a therapeutically effective amount of a particle or a pharmaceutical formulation according to the invention and exposing cancer cells to light.

A twelfth aspect of the invention provides a method of treating or repressing a cancer, said method comprising administering in a subject in need thereof a therapeutically effective amount of a particle or a pharmaceutical formulation according to the invention and exposing cancer cells to light.

A thirteenth aspect of the invention provides a method of diagnosis of a cancer cell, said method comprising the following steps:
a) administering in a subject in need thereof a detectably effective amount of a particle or a pharmaceutical formulation according to the invention;
b) exposing the site of investigation of the subject's body to light.

### Description of the figures

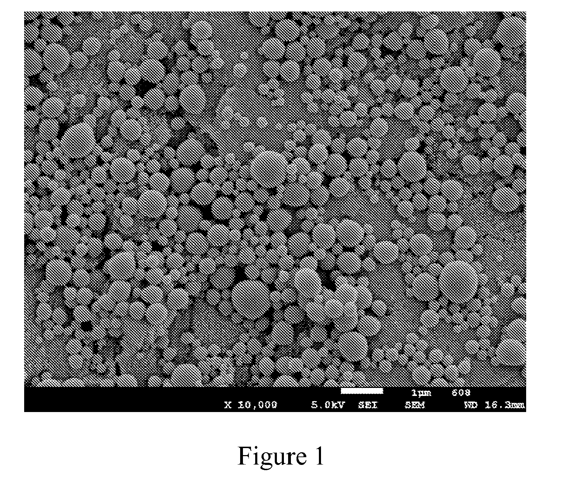

**Figure 1** represents a scanning electron microscopy (SEM) image of HAL Butbs laden nanoparticles as described in Example 2.
**Figure 2** represents fluorescence intensity in arbitrary units [a.u.] at 635 nm after background subtraction in human T24 cells incubated with HAL free salts and its corresponding nanoparticles as a function of concentration and time (hours) as described in Example 3. **A&B:** HAL HCl free and encapsulated salt respectively; **C & D:** HAL Butbs free and encapsulated salt, respectively; **E&F:** HAL Hexbs free and encapsulated salt, respectively.
**Figure 3** represents fluorescence time profile in human T24 cells exposed to HAL salts in their free form (A) and encapsulated into nanoparticles (B) as described in Example 3.

### Detailed description of the invention

The term "polymer," as used herein, is given its ordinary meaning as used in the art, i.e., a molecular structure comprising one or more repeating units (monomers), connected by covalent bonds. The repeating units may all be identical, or in some cases there may be more than one type of repeating unit present within the polymer. In some cases, the polymer can be biologically derived, i.e., a biopolymer. In some cases, additional moieties may also be present in the polymer, for example biological moieties such as amino acids (e.g. lysine) or sugars (e.g. saccarides). If more than one type of repeating unit is present within the polymer, then the polymer is said to be a "copolymer." The repeating units forming the said copolymer may be arranged in any fashion. For example, the repeating units may be arranged in a random order, in an alternating order, or as a block copolymer, i.e., comprising one or more regions each comprising a first repeating unit (e.g., a first block), and one or more regions each comprising a second repeating unit (e.g., a second block), etc. Block copolymers may have two (a diblock copolymer), three (a triblock copolymer), or more numbers of distinct blocks.

The term "biocompatible", refers to an agent that does not induce an adverse response when inserted or injected into a living subject, for example, without inducing significant inflammation and/or acute rejection of the agent by the immune system, for instance, via a T-cell response. As opposed, a non-biocompatible material implanted/administered into a subject provokes an immune response in the subject that can be severe enough such that the rejection of the material by the immune system cannot be adequately controlled, and often is of a degree such that the material must be removed from the subject. Biocompatibility can be determined for example by exposing a polymer to cells *in vitro* where biocompatible polymers are polymers that typically will not result in significant cell death at moderate concentrations. Biocompatibility may be assayed as described in Williams, 2008, Biomaterials, 29: 2941-2953 or Ignatius et al., 1996, Biomaterials, 17: 831-9*,* for example. Non-limiting examples of biocompatible polymers that may be useful in various embodiments of the present invention include poly(D,L-lactic acid) (PLA), poly(lactic-co-glycolic acid), polycaprolactone, chitosan, polysaccharides, proteins (e.g. BSA), polyacrylates/-methacrylates, cellulose, alginate, polyanhydrides, polydioxanes (e.g. PTMC), polyorthoesters, polyamides, PTFE, collagen, hyaluronic acid, or copolymers or derivatives including these and/or other polymers. In one aspect, biocompatible polymers may be biodegradable.

The term "biodegradable" as applied to particles of the invention means particles comprising biodegradable polymers, i.e. polymers which are degradable within a physiological environment, such as within the body, either enzymatically or non-enzymatically to produce biocompatible or non-toxic by-products which can be further metabolized or excreted via normal physiological pathways (for example, polylactide may be hydrolyzed to form lactic acid, polyglycolide may be hydrolyzed to form glycolic acid). The term "5-ALA ester" includes esters of 5-Aminolevulinic acid such as those described in *WO 96*/*28412, WO 02*/*10120 or Fotinos et al., 2006, above* and derivatives thereof. Exemplary 5-ALA esters include hexyl aminolevulinate and methyl aminolevulinate.

The term "5-ALA ester derivative" refers to any derivative of a 5-ALA ester, which is capable of providing directly or indirectly, the activity disclosed herein.

The term "cancer" includes metastatic and non-metastatic cancers such as colon cancer, rectal cancer, breast cancer, mama carcinoma, lymphoma, brain cancer, ovarian cancer, non-small cell lung cancer, colorectal carcinoma, glioblastoma, gastric, bronchus cancer, pancreatic cancer, urinary bladder cancer, hepatic cancer, non-melanoma skin cancer, oesophageal cancer, oral cancer, duodenal cancer, cervix cancer, uterus cancer, kidney cancer and prostate cancer. "Cancer cells" can occur in form of tumor tissue, be present as isolated cells in a subject (e.g., leukemia cells), or be cell lines derived from a cancer.

As used herein, "treatment" and "treating" and the like generally mean obtaining a desired pharmacological and physiological effect. The term "treatment" as used herein covers any treatment of a disease in a mammal, particularly a human, and includes inhibiting the disease, i.e., arresting its development; or relieving the disease, i.e. causing regression of the disease and/or its symptoms or conditions such as tumor growth arrest or tumor regression. Diseases or disorders, which may be treated or diagnosed according to the present invention include any malignant, pre-malignant and non-malignant abnormalities responsive to photochemotherapy.

The term "subject" as used herein refers to mammals. For examples, mammals contemplated by the present invention include human, primates, domesticated animals such as cattle, sheep, pigs, horses, laboratory rodents and the like.

The term "effective amount" as used herein refers to an amount of at least one particle or a pharmaceutical formulation thereof according to the invention that elicits the biological or medicinal response in a tissue, system, animal or human that is being sought. In one embodiment, the effective amount is a "therapeutically effective amount" for the alleviation of the symptoms of the disease or condition being treated. Typically, an effective amount can be used to inhibit the growth of cancer cells, e.g., prostate cancer cells, i.e. any slowing of the rate of cancer cell proliferation and/or migration, arrest of cancer cell proliferation and/or migration, or killing of cancer cells, such that the rate of cancer cell growth is reduced in comparison with the observed or predicted rate of growth of an untreated control cancer cell. The term "inhibits growth" can also refer to a reduction in size or disappearance of a cancer cell or tumor, as well as to a reduction in its metastatic potential. Preferably, such an inhibition at the cellular level may reduce the size, deter the growth, reduce the aggressiveness, or prevent or inhibit metastasis of a cancer in a patient. Those skilled in the art can readily determine, by any of a variety of suitable indicia, whether cancer cell growth is inhibited.

The term "efficacy" of a treatment according to the invention can be measured based on changes in the course of a disease in response to a use or a method according to the invention. The efficacy of a treatment of a cancer according to the invention can be measured by a reduction of tumor volume, and/or an increase of progression free survival time and/or increased health and well-being of the subject (e.g. repressing a cancer). Inhibition of cancer cell growth may be evidenced, for example, by arrest of cancer cells in a particular phase of the cell cycle, e.g., arrest at the G2/M phase of the cell cycle. Inhibition of cancer cell growth can also be evidenced using well known imaging methods such as magnetic resonance imaging, computerized axial tomography and X-rays. Cancer cell growth can also be determined indirectly, for example by determining the levels of circulating carcinoembryonic antigen, prostate specific antigen or other cancer- specific antigens that are correlated with cancer cell growth.

The term "detectably effective amount" as used herein refers to an amount of at least one particle or a pharmaceutical formulation thereof according to the invention that elicits a detectable fluorescence emission response in a cancer cell from a tissue, system, animal or human that is being investigated. In one embodiment, the "detectably effective amount" is an amount necessary for the detection of a fluorescence emission signal between about 600 and about 750 nm in a cancer cell from a tissue, system, animal or human that is being investigated induced by the exposure of said tissue, system, animal or human to an excitation signal at about 300 and about 700 nm.

The term "C₁-C₂₀ alkyl" when used alone or in combination with other terms, comprises monovalent straight chain or branched alkyl groups having 1 to 20 carbon atoms. This term is exemplified by groups such as ethyl, n-butyl, sec-butyl, iso-butyl, tert-butyl, n-pentyl, 1-ethylpropyl, 2-methylbutyl, 3-methylbutyl, 2,2-dimethylpropyl, n-hexyl, 2-methylpentyl, 3-methylpentyl, 4-methylpentyl, n-heptyl, 2-methylhexyl, 3-methylhexyl, 4-methylhexyl, 5-methylhexyl, n-octyl and the like. Particularly, those include C₁-C₆ alkyl, which, by analogy, refer respectively to monovalent straight chain or branched alkyl groups having 1 to 6 carbon atoms.

The term "C₂-C₂₀ alkenyl" when used alone or in combination with other terms, comprises a straight or branched alkenyl chains with a carbon number of 2-20 having any available number of double bonds in any available positions, and the configuration of the double bond may be the (E) or (Z) configuration. This term is exemplified by groups such as vinyl, allyl, isopropenyl, 1-propenyl and the like.

The term "C₂-C₂₀ alkynyl" when used alone or in combination with other terms, comprises a straight or branched alkynyl chains with a carbon number of 2-20 having any available number of triple bonds in any available positions. This term is exemplified by groups such as ethynyl (-C≡CH), 1-propynyl, 2-propynyl (propargyl: -CH₂C≡CH), 2-butynyl, 2-pentene-4-ynyl, and the like.

The term "aryl" refers to an unsaturated aromatic carbocyclic group of from 6 to 20 carbon atoms having a single ring (e.g., phenyl) or multiple condensed rings (e.g., indenyl, naphthyl). In a particular embodiment, aryl refers to aromatic carbocyclic group of from 6 to 12 carbons, typically 6 to 10 carbons. Aryl include phenyl, naphthyl, anthryl, phenanthrenyl and the like.

The term "heteroaryl" refers to a monocyclic heteroaromatic, or a bicyclic or a tricyclic fused-ring heteroaromatic group.

The term "alkoxy" refers to the group -O-R where R includes "C₁-C₆ alkyl", "aryl", "heteroaryl", "aryl C₁-C₆ alkyl" or "heteroaryl C₁-C₆ alkyl". Preferred alkoxy groups include for example, methoxy, ethoxy, phenoxy and the like.

Unless otherwise constrained by the definition of the individual substituent, all the above substituents shoud be understood as being all optionally substituted.

Unless otherwise constrained by the definition of the individual substituent, the term "substituted" refers to groups substituted with from 1 to 5 substituents selected from the group consisting of "C₁-C₆ alkyl," "C₂-C₆ alkenyl," "C₂-C₆ alkynyl," "alkoxy," "halogen," amino, cyano, hydroxy, mercapto, nitro, and the like.

The term "halogen" refers to fluoro, chloro, bromo and iodo atoms.

The term "pharmaceutical formulation" refers to preparations which are in such a form as to permit biological activity of the active ingredient(s) to be unequivocally effective and which contain no additional component which would be toxic to subjects to which the said formulation would be administered.

According to a further aspect, the invention provides a particle for systemic administration in a human or animal host, said particle comprising a biocompatible polymer and salt of a 5-Aminolevulinic acid ester, wherein the salt is selected from an aryl sulfonate salt of Formula (I), an aryl phosphate salt of Formula (II) and an aryl nitrate salt of Formula (III): 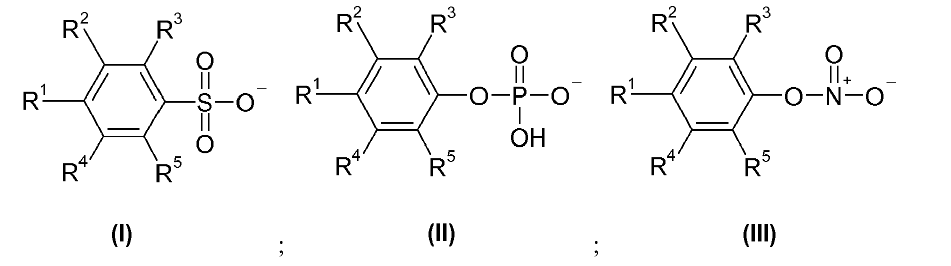 wherein R¹, R², R³, R⁴ and R⁵ are independently selected from H, optionally substituted C₁-C₂₀ alkyl, optionally substituted C₂-C₂₀ alkenyl, optionally substituted C₂-C₂₀ alkynyl, optionally substituted alkoxy, optionally substituted amino, halogen, hydroxyl and nitro, wherein at least one of R¹, R², R³, R⁴ and R⁵ is not H or mixtures thereof.

According to a further aspect, the invention provides a particle according to the invention wherein the salt is an aryl sulfonate salt of Formula (I), wherein R¹, R², R³, R⁴ and R⁵ are as defined in the detailed description.

Examples of 5-Aminolevulinic acid esters include those of Formula (IV):

R⁷R⁸N-CH₂COCH₂-CH₂CO-OR⁶ **(IV)**

wherein R⁶ is selected from optionally substituted C₁-C₂₀ alkyl, optionally substituted C₂-C₂₀ alkenyl and C₂-C₂₀ alkynyl; R⁷ and R⁸ are independently selected from H and optionally substituted C₁-C₂₀ alkyl. When R⁶ is a substituted C₁-C₂₀ alkyl, one or more substituents are either attached to the alkyl group and/or interrupt the alkyl group. For example, suitable substituents that are attached to the alkyl group may be selected from hydroxy, alkoxy, acyloxy, alkoxycarbonyloxy, amino, aryl, nitro, oxo, fluoro, -SR⁹, -NR⁹R¹⁰ and -PR⁹R¹⁰, wherein R⁹ and R¹⁰ are independently selected from H and C₁₋₆ alkyl. Suitable substituents that interrupt the alkyl group may be selected from -O-, -NR⁹-, -S- or -PR⁹.

In a particular embodiment, R⁷ is H.

In another particular embodiment, R⁸ is H.

In another particular embodiment, R⁶ is optionally substituted C₁-C₆alkyl.

In a further particular embodiment, R⁶ is hexyl.

In another further particular embodiment, R⁶ is methyl.

In a further particular embodiment, the invention provides a particle according to the invention wherein the 5-ALA ester is hexyl aminolevulinate.

In another further particular embodiment, the invention provides a particle according to the invention wherein the 5-ALA ester is methyl aminolevulinate.

5-ALA esters for use in the context of the invention may be prepared by any conventional procedure available in the art, e.g. as described in WO 96/28412 and WO 02/10120. Briefly, 5-ALA esters may be prepared by reaction of 5-ALA with an appropriate alcohol in the presence of a catalyst, e.g. an acid.

### Particles

According to a further aspect, the systemic administration is selected from oral and parenteral administration.

According to another further aspect, is provided a particle according to the invention, wherein R¹ is an optionally substituted C₂-C₂₀ alkyl.

According to another further aspect, is provided a particle according to the invention, wherein R¹ is an optionally substituted C₂-C₆ alkyl.

According to another further aspect, is provided a particle according to the invention, wherein R¹ is selected from optionally substituted ethyl, optionally substituted butyl, optionally substituted hexyl, optionally substituted octyl and optionally substituted dodecyl. According to another further aspect, is provided a particle according to the invention, wherein R², R³, R⁴ and R⁵ are H.

According to another further aspect, is provided a particle according to the invention, wherein the aryl sulfonate is selected from ethyl benzene sulfonate (Etbs), butyl benzene sulfonate (Butbs), hexyl benzene sulfonate (Hexbs), octyl benzene sulfonate (Octbs) and dodecyl benzene sulfonate (DBS).

According to another further aspect, is provided a particle according to the invention, wherein the 5-Aminolevulinic acid ester is selected from methyl aminolevulinate and hexyl aminolevulinate.

A wide variety of polymers and methods for forming particles therefrom are known in the art of drug delivery, such as those described in Quintanar-Guerrero et al., 1998, Drug Delivery in Industrial Pharmacy, 1113-1128*.* Polymers used for forming particles according to the invention can be natural or unnatural (synthetic) polymers. Those polymers can be homopolymers or copolymers comprising two or more monomers. In terms of sequence, copolymers can be random, block, or comprise a combination of random and block sequences. Typically, polymers in accordance with the present invention are organic polymers.

According to an aspect, polymers may be polyesters, including copolymers comprising lactic acid and glycolic acid units, such as poly(lactic acid-co-glycolic acid) and poly(lactide-co-glycolide), collectively referred to herein as "PLGA"; and homopolymers comprising glycolic acid units, referred to herein as "PGA," and lactic acid units, such as poly- L-lactic acid, poly-D-lactic acid, poly-D,L-lactic acid, poly-L-lactide, poly-D-lactide, and poly- D,L-lactide, collectively referred to herein as "PLA." Exemplary polyesters include, for example, Poly lactic acid (PLA), poly(lactic-co-glycolic acid) (PLGA), Polycaprolactone (PCL), polyhydroxy valerate, polyhaydroxy butyrate, polyorthoesters, and derivatives thereof.

For example, disclosed here is an exemplary particle that includes about 30 to about 95 weight percent polymer, typically about 70 to about 90 weight percent polymer. Exemplary polymers can include a number average molecular weight of about 0.5 to about 300 10³ kDa, typically from about 20 to about 500 kDa (e.g. about 150 kDa). According to an aspect, using two or more different polymers (e.g. copolymers, block copolymers) in different ratios and producing particles from the polymers (e.g., copolymers, e.g., block copolymers), properties such as biocompatibility and/or ability to control immunogenicity and/or organ/tumor specificity of the resulting particles may be assayed by methods known to the skilled person such as those described in Basarkar et al., 2007, International Journal of Pharmaceutics, 343: 247-254 or Zeisser-Labouebe et al., 2009, Nanomedicine,4: 135.143*.*

In a particular aspect, the particles comprise a biodegradable polymer.

According to another further aspect, is provided a particle according to the invention, wherein the biocompatible polymer is PLA.

Particles according to the invention include micro or nanoparticles having a substantially spherical (i.e. particles generally appearing to be spherical) or non-spherical configuration. For instance, substantially spherical particles, upon swelling or shrinkage, may adopt a non-spherical configuration.

According to another further aspect, is provided a particle according to the invention, wherein the particle has a diameter from about 1 nm to about 10 µm, typically a diameter between 50 nanometers and 900 nanometers.

According to another further aspect, is provided a particle according to the invention for use as a medicament.

According to another further aspect, is provided a particle according to the invention for use in the photodiagnostic imaging (e.g. by fluorescence photodetection) of cancer. Typically, particles according to the invention include about 1 to about 70 weight percent of 5-ALA ester salt, for example about 10 to about 30 weight percent of 5-ALA ester salt. In a particular aspect, the particles may present controlled release properties, e.g., may be capable of delivering an amount of 5-ALA ester salt to a patient, e.g., to specific site in a patient, over an extended period of time, e.g. over 24 hours or more. For example, in contrast to systemic delivery of a free 5-ALA ester salt, the systemic delivery of particles disclosed herein may substantially prevent the agent from killing healthy cells or to induce severe side effects in a subject. Additionally, disclosed particles may allow the induction of higher amounts of photoactive porphyrin and/or the long lasting of induced high photoactive porphyrin contents (as compared to an effective amount of a free 5-ALA ester salt). Those properties confer to those particles and formulations thereof reduced undesirable side effects and increased efficacy as compared to agents commonly used in traditional chemotherapy, thus an increased therapeutic index.

### Formulations according to the invention

Particles disclosed herein may be combined with one or more pharmaceutical acceptable carrier(s) to form a pharmaceutical composition.

According to an embodiment, is provided a pharmaceutical formulation, said formulation comprising at least one particle according to the invention and at least one pharmaceutically acceptable carrier.

According to another embodiment, is provided particle or a formulation thereof obtainable by a process or a method according to the invention.

According to a further embodiment, the invention provides a pharmaceutical formulation for systemic administration in a mammal, typically a human mammal.

According to a further embodiment, the invention provides a pharmaceutical formulation suitable for injection in humans (e.g. intravenous).

According to another further embodiment, the invention provides a formulation according to the invention for use as a medicament.

According to another further embodiment, the invention provides a kit comprising in one or more container(s) a formulation according to the invention together with instruction of use of said formulation.

According to a further aspect, the invention provides a kit for use in a method of treating or reducing a cancer comprising: a) a first container containing a formulation according to the invention and optionally b) one or more chelating agents contained either within said first container or in a second container. According to a further aspect, the invention provides a photodynamic therapy kit further comprising a light emitting device for irradiation of light of wavelength of about 300 nm to about 800 nm, typically about 380 nm to about 660 nm. According to a further aspect, the invention provides a kit for use in a method of detection of a cancer cell comprising: a) a first container containing a formulation according to the invention and optionally b) one or more chelating agents contained either within said first container or in a second container. According to a further aspect, the invention provides a photodetection kit further comprising a light emitting device for irradiation of light of wavelength of about 300 nm to about 800 nm, typically about 380 nm to about 660 nm and a second device capable for visualizing the emitted fluorescence (typically from about 600 to about 750 nm).

According to another further embodiment, the invention provides a formulation according to the invention for the treatment of cancers (e.g. by photodynamic therapy).

According to another further embodiment, the invention provides a formulation according to the invention for the diagnosis of a cancer cell (e.g. by fluorescence photodetection). According to another further embodiment, the invention provides a formulation according to the invention further comprising a co-agent useful in the treatment of cancer, such as substances useful for treating, stabilizing, preventing, and/or delaying cancer.

Particles according to the invention may be administered as a pharmaceutical formulation which can contain one or more particles according to the invention and a pharmaceutically acceptable carrier, in any form described herein.

In a particular aspect, the formulation is adapted for delivery by repeated administration.

In another aspect, the pharmaceutical compositions may be placed separately into the form of unit dosages.

As it would be appreciated by one skilled person in this art, the carriers may be chosen based on the route of administration as described below, the location of the target tissue, the drug being delivered, the time course of delivery of the drug, etc.

Formulations of this invention may further comprise one or more pharmaceutically acceptable additional ingredient(s) such as alum, stabilizers, antimicrobial agents, buffers, coloring agents, flavoring agents, adjuvants, and the like.

Formulations of the invention, together with a conventionally employed adjuvant, carrier, diluent or excipient may be employed as liquids such as solutions, suspensions, emulsions, elixirs, or capsules filled with the same, or in the form of sterile injectable solutions. Compositions of this invention may also be formulated for parenteral administration including, but not limited to, by injection or continuous infusion. Such liquid preparations may contain additives including, but not limited to, dispersing or wetting agents, suspending agents, emulsifying agents, non-aqueous vehicles and preservatives. Injectable preparations may be prepared for example as sterile injectable aqueous or oleaginous suspensions, or emulsion in a nontoxic parenterally acceptable diluent or solvent. Among the acceptable vehicles and solvents that may be employed are water, Ringer's solution, and isotonic sodium chloride solution or other injectable carriers known in the art. The injectable formulations can be sterilized, for example, by incorporating sterilizing agents in the form of sterile solid compositions which can be dissolved or dispersed in sterile water or other sterile injectable medium prior to use. The compositions may also be provided in a powder form for reconstitution with a suitable vehicle including, but not limited to, sterile, pyrogen-free water.

Suspending agents include, but are not limited to, sorbitol syrup, methyl cellulose, glucose/sugar syrup, gelatin, hydroxyethyl cellulose, carboxymethyl cellulose, aluminum stearate gel, and hydrogenated edible fats. Emulsifying agents include, but are not limited to, lecithin, sorbitan monooleate, and acacia. Preservatives include, but are not limited to, methyl or propyl p-hydroxybenzoate and sorbic acid. Dispersing or wetting agents include but are not limited to poly(ethylene glycol), glycerol, bovine serum albumin, Tween®, Span®.

Compositions of this invention may also be formulated as a depot preparation, which may be administered by implantation or by intramuscular injection. The compositions may be formulated with suitable polymeric or hydrophobic materials (as an emulsion in an acceptable oil, for example), ion exchange resins, or as sparingly soluble derivatives (as a sparingly soluble salt, for example).

Compositions of this invention may also be formulated for inhalation, which may be in a form including, but not limited to, a solution, suspension, or emulsion that may be administered as a dry powder or in the form of an aerosol using a propellant, such as an inhaler (e.g. Turbohaler®, Spinhaler®, Diskhaler®, Easyhaler®) as described in Pharmazeutische Technologie, 9th Edition, 2000, Dt. Apotheker Verlag, Stuttgart*.* Typically, the aerosol is delivered to the airways from the patient.

Compositions of this invention may also be formulated as transdermal formulations comprising aqueous or non-aqueous vehicles including, but not limited to, creams, ointments, lotions, pastes, medicated plaster, patch, or membrane.

Compositions of this invention may also be formulated for oral administration. Solid dosage forms for oral administration include capsules, tablets, pills, powders, and granules. In such solid dosage forms, the particles of the invention are mixed with at least one inert, pharmaceutically acceptable excipient or carrier such as sodium citrate or dicalcium phosphate and/or (a) fillers or extenders such as starches, lactose, sucrose, glucose, mannitol, and silicic acid, (b) binders such as, for example, carboxymethyl cellulose, alginates, gelatin, polyvinylpyrrolidinone, sucrose, and acacia, gelatine (c) humectants such as glycerol, (d) disintegrating agents such as agar-agar, calcium carbonate, potato or tapioca starch, alginic acid, certain silicates, and sodium carbonate, polyvinylpyrrolidone (e) solution retarding agents such as paraffin, ethyl cellulose, polyacrylate/methacrylate (f) absorption accelerators such as quaternary ammonium compounds, (g) wetting agents such as, for example, cetyl alcohol and glycerol monostearate, (h) absorbents such as kaolin and bentonite clay, and (i) lubricants such as talc, calcium stearate, magnesium stearate, solid polyethylene glycols, sodium lauryl sulfate, and mixtures thereof. In the case of capsules, tablets, and pills, the dosage form may also comprise buffering agents.

In an exemplary embodiment, a pharmaceutical composition according to the invention includes a plurality of particles according to the invention, each comprising 5-ALA ester salt, about 1 to about 70 weight percent of the total polymer content, or about 5 to about 60 weight percent, or about 10 to about 50 weight percent, or about 10 to about 30 weight percent of the total polymer content of a particle, and a pharmaceutically acceptable excipient.

In one embodiment, is provided a composition according to the invention suitable for freezing wherein the composition includes particles disclosed herein and a solution suitable for freezing, e.g. a cryoprotectant to prevent the particles from aggregating upon freezing such as a sucrose, trehalose, mannitol, glucose, lactose, sorbitol, polyvinylpyrrolidone, glycerol, glycine, gelatine, maltose or fructose solution or a mixtures thereof is added to the particle suspension.

In another embodiment, is provided a composition suitable for use as a precursor for preparing tablets or preparation for compositions for inhalation.

In another embodiment, is provided a composition suitable for use in the photodiagnostic imaging of cancer (e.g. by fluorescence photodetection).

Further materials as well as formulation processing techniques and the like are set out in Part 5 of Remington's Pharmaceutical Sciences, 21st Edition, 2005, University of the Sciences in Philadelphia, Lippincott William & Wilkins*,* which is incorporated herein by reference.

### Methods of preparation of particles according to the invention

According to one aspect of the invention, is provided methods for the preparation of particles according to the invention.

In another aspect, is provided a process for the preparation of a particle for systemic administration in a human or animal host or a formulation thereof comprising the steps of:
(i) providing a solution comprising a 5-ALA ester salt selected from an aryl sulfonate, an aryl phosphate salt and an aryl nitrate salt and one or more biocompatible polymer;
(ii) providing a solvent in which the said biocompatible polymer is not soluble;
(iii) contacting the solution provided under step (i) with the solvent provided under step (ii);
(iv) membrane filtering the mixture obtained under step (iii);
(v) collecting the particles filtered under step (iv).

According to a further aspect, the invention provides a process for the preparation of a particle according to the invention, wherein the process further comprises a step (ia) wherein a surfactant is added to the solution provided under step (i).

According to another further aspect, the invention provides a process for the preparation of a particle according to the invention, wherein the process further comprises a step (ia) wherein a saturated or unsaturated fatty acid (such as myristic acid, caproic acid, caprylic acid, capric acid, oleic acid, palmic acid, palmitolic acid, lauric acid, stearic acid, undecylenic acid and nonanoic acid) is added to the solution provided under step (i).

According to another further aspect, the invention provides a process for the preparation of a particle according to the invention, wherein the process further comprises a step (ia) wherein lauric acid is added to the solution provided under step (i).

According to a further aspect, the invention provides a process for the preparation of a particle according to the invention, wherein the process further comprises a step (via) sterilizing the collected particles under step (v), typically by γ-irradiating the collected particles under step (v) or by exposing the particles collected under step (v) to an antimicrobial solution.

For example, the solution comprising one or more polymers comprises a water miscible organic solvent (e.g. acetonitrile, tetrahydrofuran, acetone, formamide, dimethylformamide, pyridines, dioxane, dimethysulfoxide, etc.) and the solvent in which the polymer is not soluble comprises an aqueous liquid (e.g., water, or water containing dissolved salts). Alternatively, particles may be formed by emulsion evaporation, emulsion diffusion or salting out such as described in *Quintanar-Guerrero et al., 1998, above.*

The control of such particle formation can be readily optimized by one of ordinary skill in the art using routine experimentation such as SEM, Transmission electron microscopy (TEM), Atomic force microscopy (AFM), laser diffractometry, and photon correlation spectroscopy.

5-ALA esters may be prepared as described in *WO 96*/*28412; WO 02*/*10120;* Fotinos et al., 2006, Photochemistry and Photobiology, 82: 994-1015 or Kloek et al., 1996, Photochemistry and Photobiology, 64: 994-1000*.* In particular, HAL may be prepared as described in Kloek et al., 1998, Photochem Photobiol, 67: 150-154 *or* Lange et al., 1999, Br. J. Cancer, 80:185-193*.* Salts of 5-ALA esters may be prepared according to methods known in the art such as described in Gray et al., 1955, Journal of Organic Chemistry, 20: 511-524 *and in WO 2005*/*092838.*

### Mode of administration

Formulations of this invention may be administered in any manner including orally, parenterally, vaginally, rectally, intranasally, intraarticularily, intravesically, intraauricularly, topically or by inhalation or combinations thereof.

In a particular embodiment, the particles of the present invention are administered to a subject in need thereof systemically, e.g. by i.v. infusion or injection.

In a particular embodiment, the particles of the present invention are orally administered to a subject in need thereof (e.g. as capsule, tablet, or suspension).

Parenteral administration includes, but is not limited to, intravenous, intra-arterial, intraperitoneal, subcutaneous and intramuscular. According to a particular aspect, the compositions of this invention are administered in the form of an implant, which allows slow release of the compositions as well as a slow controlled i.v. infusion.

### Uses and methods according to the invention

Photodynamic therapy based on the systemic administration of particles according to the invention and formulations thereof, for the treatment of cancers uses the body's own biosynthetic ability to form the endogenous intracellular chromophore protoporphyrin IX (PpIX). In Photodynamic diagnosis (PDD), the strong fluorescence of this chromophore is induced by an excitation signal at 380-660 nm which yields a strong emission in the range 600-750 nm, enabling detection of the tissue in which PpIX is accumulated.

According to one aspect, the invention provides a method of treating or repressing a cancer, said method comprising administering in a subject in need thereof a therapeutically effective amount of a pharmaceutical formulation according to the invention and exposing cancer cells to light. Typically, in a method of treatment according to the invention, protoporphyrin IX intracellular accumulation into cancer cells is triggered by the administration of a formulation according to the invention and cancer cells are exposed to a light having a suitable wavelength (e.g. white light, for example light in the wavelength region 300-800 nm, typically light in the wavelength region 380-660 nm) in order to activate protoporphyrin IX and convert it into a cytotoxic form thereof.

According to another aspect, the invention provides a method of diagnosis of a cancer cell, said method comprising the following steps:
a) administering in a subject in need thereof a detectably effective amount of a pharmaceutical formulation according to the invention;
b) exposing the site of investigation of the subject's body to light.

Typically, in a method of diagnosis according to the invention, intracellular protoporfyrin IX accumulation at the site of investigation is triggered by the administration of a formulation according to the invention and the site of investigation of the subject's body is then exposed to a light having a suitable wavelength to excite the fluorescence of protoporphyrin IX (e.g. light in the wavelength region 300-800 nm, typically blue light in the wavelength region 350-440 nm) and measure the triggered fluorescence (e.g. in the wavelength region 550-750 nm) which may be used to visualize the size, extent and situation of an abnormality or disorder.

According to a further embodiment, the method of diagnosis according to the invention comprises the further steps of:
c) ascertaining the level of fluorescence induced under step b);
d) comparing the level of fluorescence measured under step c) to control levels.

According to a further embodiment, the method of diagnosis according to the invention wherein the level of fluorescence ascertained under step c) is measured at a wavelength equal to or between about 620 and about 640 nm.

The abnormality or disorder thus identified or confirmed at the site of investigation may then be treated through alternative therapeutic techniques e.g. surgical or chemical treatment, or by a method of treatment of the invention by continued build-up of fluorescence or through further application of formulations of the invention at the appropriate site. It will be appreciated that diagnostic techniques may require lower levels of fluorescence for visualization than used in therapeutic treatments.

Methods for irradiation of different areas of the body, e. g. by lamps or lasers are well known in the art (*e.g.* Van den Bergh, 1986, Chemistry in Britain, p. 430-439). For inaccessible regions this may conveniently be achieved using optical fibres as described in Hasselgren et al., 1990, Applied Optics, 29: 44481-44488*.*

According to a further aspect, the invention provides a method of treating or repressing a cancer or a method of diagnosis of a cancer cell, wherein cancer cells are only efficiently accessible systemically such as in the case of colon cancer, rectal cancer, breast cancer, mama carcinoma, lymphoma, brain cancer, ovarian cancer, non-small cell lung cancer, colorectal carcinoma, glioblastoma, gastric, bronchus cancer, pancreatic cancer, urinary bladder cancer, hepatic cancer, non-melanoma skin cancer, oesophageal cancer, oral cancer, duodenal cancer, cervix cancer, uterus cancer, kidney cancer and prostate cancer.

According to a further aspect of the invention, the invention provides a method of treating or repressing a cancer or a method of diagnosis of a cancer cell, wherein particles or formulation thereof according to the present invention are administered parenterally and then, between about 1 minute to about 48 hours, typically between about 30 minutes and about 24 hours after administration the cells/the site of investigation are then illuminated with visible light, typically in the region between about 350 and about 750 nm, allowing for said diagnosis or therapy.

According to a further aspect of the invention, the invention provides a method of treating or repress a cancer or a method of diagnosis of a cancer cell, wherein particles or formulation thereof according to the present invention are orally administered (e.g. as capsule or tablet).

According to a further aspect of the invention, is provided a use of a particle or a formulation according to the invention for the treatment of a cancer.

According to a further aspect of the invention, is provided a use of a particle or a formulation according to the invention for the preparation of a pharmaceutical formulation for treatment of a cancer.

According to a further aspect of the invention, is provided a use of a particle or a formulation according to the invention for the diagnosis of a cancer cell.

According to a further aspect of the invention, is provided a use of a particle or a formulation according to the invention for the preparation of a pharmaceutical formulation for diagnosis of a cancer cell.

According to a further aspect, are provided uses and methods according to the invention wherein a pharmaceutical formulation according to the invention is to be systemically administered.

The dosage administered, as single or multiple doses, to an individual will vary depending upon a variety of factors, including pharmacokinetic properties, patient conditions and characteristics (sex, age, body weight, health, size), extent of symptoms, concurrent treatments, frequency of treatment and the effect desired.

### Combinations

According to one embodiment of the invention, the particles according to the invention and pharmaceutical formulations thereof can be administered alone or in combination with a co-agent useful in the treatment of cancer, in particular substances useful for treating, stabilizing, preventing, and/or delaying cancer such as substances used in conventional chemotherapy directed against solid tumors and for control of establishment of metastases or any other molecule that act by triggering programmed cell death e.g. for example a co-agent selected from angiogenesis inhibitors (e.g. anti-VEGF agents, anti-PDGF agents), immunotherapy agents (e.g. recombinant cytokines, interferones, interleukin, recombinant antibodies such as herceptin®) and chemotherapy agents (e.g. cisplatin, paclitaxel, methotrexate, 5-fluoruracil, Gemcitabin, Vincristin, Vinblastin, Doxorubicin).

According to another aspect, the particles according to the invention and pharmaceutical formulations thereof be administered alone or in combination with a co-agent useful in improve the efficacy of PDT such as photosensitizing agents (e.g.psoralens, porphyrins such as Photofrin®, chlorins and the phthalocyanins) or with other active components which may enhance the photochemotherapeutic effect such as chelating agents (e.g. desferrioxamine).

According to one embodiment of the invention, the particles according to the invention and pharmaceutical formulations thereof can be administered alone or in combination with a co-agent useful in the detection of a cancer cell, typically agents enhancing the detectivity of fluorescence such as Photofrin®.

The invention encompasses the administration of particles according to the invention or pharmaceutical formulations thereof, wherein the particles according to the invention or the pharmaceutical formulation thereof are administered to an individual prior to, simultaneously or sequentially with other therapeutic regimens or co-agents useful in the treatment of cancers or co-agents useful in the detection of a cancer cell (e.g. multiple drug regimens), in a therapeutically effective amount. Particles according to the invention or pharmaceutical formulations thereof, that are administered simultaneously with said co-agents can be administered in the same or different composition(s) and by the same or different route(s) of administration.

### Patients

In an embodiment, patients according to the invention are patients suffering from a disease or disorder including any malignant, pre-malignant and non-malignant abnormalities responsive to photochemotherapy, including, but not limited to, tumors or other hyperproliferative conditions such as cancers, skin disorders such as psoriasis, skin cancer, or actinic keratosis, infectious diseases (e.g. viral, bacterial, fungal infections), inflammatory diseases like Morbus Crohn, arthritis and rheumatoid arthritis, Barrett's oesophagus or arterial restenosis.

In a particular embodiment, patients according to the invention are suffering from a cancer. In another particular embodiment, patients according to the invention are suffering from bladder, kidney, colon, breast, brain, ovarian, skin, or prostate cancer.

In another particular embodiment, patients according to the invention are susceptible to suffer from a cancer or to present a cancer cell in their tissues.

References cited herein are hereby incorporated by reference in their entirety. The present invention is not to be limited in scope by the specific embodiments and drawings described herein, which are intended as single illustrations of individual aspects of the invention, and functionally equivalent methods and components are within the scope of the invention. The examples illustrating the invention are not intended to limit the scope of the invention in any way.

### EXAMPLES

### GENERAL PROCEDURES & CONDITIONS

The following studies are conducted to support the high loading and encapsulation efficiencies, the high efficiency in inducing porphyrins in cancer cells and the safety of the nanoparticles according to the invention. Since systemic administration of free 5-ALA esters has shown to cause acute systemic toxicity, this study is of great importance for anticipating beneficial effects of particles and formulations thereof according to the invention in clinical use.

The following abbreviations refer respectively to the definitions below:
**min** (minute), **ml** (milliliter), **mM** (millimolar), **nm** (nanometers), BUTBS (butyl benzene sulfonate), **DBS** (dodecyl benzene sulfonate), **DMEM** (Dulbecco's Modified Eagle Medium), **DSC** (Differential scanning calorimetry), **ETBS** (Ethyl benzene sulfonate), **HAL** (hexyl aminolevulinate), **HBSS** (Hank's Buffered Salt Solution), **HEXBS** (hexyl benzene sulfonate), **IU** (International Units), **NMR** (Nuclear magnetic resonance), **MES** (Mesylate), **OCTBS** (octyl benzene sulfonate), **PLA** (Polylactic acid), **PVAL** (Poly(vinyl alcohol)), **SAL** (salicylilate), **SEM** (Scanning electron microscopy).

### Example 1: Preparation of HAL salts

Salts of the 5-ALA ester, hexyl aminolevulinate (HAL), were prepared, when not commercially available according to a procedure as described by *Gray et al., 1955, above.* The following aryl sulfonate salts according to Formula (I) were prepared: Etbs, Butbs, Hexbs, Octbs and DBS.

For the sake of comparison, the following salts were prepared as described above: Methane sulfonate (MES), SAL, Dodecylsulfate (DDS) and Bis(2-ethylhexyl) sulfosuccinate, (AOT) and hydrochloride (HCl).

To a solution of HAL (synthesized as disclosed in *Lange et al., 1999, above*) in ethyl acetate and water an equivalent amount of corresponding aryl sulfonate salt according to Formula (I) was added. After vigorous stirring, the phases were separated by centrifugation and the aqueous phase was washed twice with ethyl acetate. The aqueous phase was then discarded. After evaporation of ethylacetate the desired HAL salt was dried under vacuum and characterized by ¹H NMR (Gemini 300, 300MHz) in DMSO as described below.

### Procedure A

To 100 mg HAL (synthesized as disclosed in *Lange et al., 1999, above*) and the equimolar amount of sodium aryl sulfonate were added 4 ml ethyl acetate and 3 ml water. After vigorous stirring, the organic phase was removed and the water phase extracted twice with 4 ml ethyl acetate. The combined organic phases were washed with 2 ml water. After centrifugation and evaporation of the organic solvent, the obtained compound was dried under vacuum.

### Procedure B

To 100 mg HAL and the equimolar amount of aryl sulfonic acid were added 84 mg of NaHCO₃, 4 ml ethyl acetate and 3 ml water. After vigorous stirring, the organic phase was removed and the water phase extracted twice with 4 ml ethyl acetate. The combined organic phases werewashed with 2 ml water. After centrifugation and evaporation of the organic solvent, the obtained compound was dried under vacuum.

Salt equivalent (in mg) (and acid equivalent for C12) for 100 mg HAL:

**Table 1**

| | **C2** | **C4** | **C6** | **C8** | **C12** |
|---|---|---|---|---|---|
| **Salt [mg]** | 82 | 94 | 106 | 116 | 129 |
| **Na HCO3 [mg]** | 0 | 0 | 0 | 0 | 84 |

### NMR Monitoring

Instrument: Gemini 300, 300MHz, Solvent: D₆-DMSO

J= coupling constant; s=singlet; d=doublet; t= triplet; q= quadruplet; m= multiplet).

### Mesylate:

H¹ NMR δ (D₆-DMSO) 0.86 (t, J= 6Hz, 3H), 1.20 -1.35 (m, 6H), 1.45-1.60 (m, 2H), 2.30 (s, 3H), 2.55 (t, J=6Hz, 2H), 3.99 (s, 2H), 4.00 (t, J=7Hz, 2H), 8.02 (s, 3H) ppm.

### Tosylate

H¹ NMR δ (D₆-DMSO) 0.87 (t, J=6Hz, 3H), 1.20-1.35 (m, 8H), 1.45-1.60 (m, 2H), 2.28 (s, 3H9, 2.55 (t, J=6Hz, 2H), 2.79 (t, J= 6Hz, 2H), 3.96 (s,2H), 4.00 (t, J= 7Hz, 2H), 7.11 (d, J=8Hz, 2H), 7.47 (d, J=8Hz, 2H), 7.94 (s, 3H) ppm.

### Ethylbenzene sulfonate

H¹ NMR δ (D₆-DMSO) 0.86 (t, J=6Hz, 3H), 1.16 (t, J= 7Hz, 3H), 1.20-1.3 (m, 6H), 1.50-1.65 (m, 2H), 2.57 (t, J= 6Hz, 2H), 2.58 (q, J= 6Hz, 2H), 2.79 (t, J= 8Hz, 2H), 8.01 (s, 3H) ppm.

### Butyl benzene sulfonate

H¹ NMR δ (D₆-DMSO) 0.80-0.95 (m, 6H), 1.20-1.3 (m, 10H), 1.45-1.65 (m, 4H), 2.56 (t, J= 6Hz, 2H), 2.79 (t, J= 6Hz, 2H), 3.99 (s, 2H), 4.00 (t, J=7Hz, 2H), 7.11 (d, J=8Hz, 2H), 7.48 (d, J= 8Hz, 2H), 7.92 (s, 3H) ppm.

### Hexylbenzene sulfonate

H¹ NMR δ (D₆-DMSO) 0.80-0.95 (m, 6H), 1.20-1.3 (m, 14H), 1.50-1.60 (m, 4H), 2.56 (t, J= 6Hz, 2H), 2.79 (t, J=6Hz, 2H), 3.97 (s, 2H), 7.11 (d, J=8Hz, 2H), 7.48 (d, J=8Hz, 2H), 8.01 (s, 3H) ppm.

### Octylbenzene sulfonate

H¹ NMR δ (D₆-DMSO) 0.80-0.95 (m, 6H), 1.20-1.4 (m, 18H), 1.45-1.60 (m, 4H), 2.56 (t, J= 6Hz, 2H), 2.56 (t, J= 6Hz, 2H), 2.79 (t, J= 6Hz, 2H), 3.98 (s, 2H), 4.00 (t, J= 7Hz, 2H), 7.11 (d, J= (Hz, 2H), 7.49 (d, J=8Hz, 2H), 7.93 (s, 3H) ppm.

### Dodecyl benzene sulfonate

H¹ NMR δ (D₆-DMSO) 0.80-0.95 (m, 6H), 1.15-1.4 (m, 26H), 1.45-1.65 (m, 4H), 2.56 (t, J= 6Hz, 2H), 2.80 (t, J= 6Hz, 2H), 3.98 (s, 2H), 4.01 (t, J= 7Hz, 2H), 7.09 (d, J= 8Hz, 2H), 7.50 (d, J= 8Hz, 2H), 7.99 (s, 3H) ppm.

### Octadecyl benzene sulfonate

H¹ NMR δ (D₆-DMSO) 0.80-0.90 (m, 6H), 1.15-1.4 (m, 38H), 1.45-1.60 (m, 4H), 2.56 (t, J=6Hz, 2H), 2.79 (t, J=6Hz, 2H), 3.96 (s, 2H), 4.00 (t, J=7Hz, 2H), 7.11 (d, J=8Hz, 2H), 7.49 (d, J=8Hz, 2H), 7.91 (s, 3H) ppm.

All prepared HAL aryl sulfonate salts (e.g. ethyl benzene sulfonate, butyl benzene sulfonate, hexyl benzene sulfonate, octyl benzene sulfonate, and dodecyl benzene sulfonate) showed a one to one ratio between the HAL cation and the (sulfonate) anion by NMR.

### Example 2: Preparation of HAL laden particles

Several methods and polymers as described in Zeisser et al., 2006, European Journal of Pharmaceutics and Biopharmaceutics, 71: 207-213*; Quintanar-Guerrero et al., 1998, above;* Cirstoiu-Hapca et al., 2007, International Journal of Pharmaceutics, 331: 190-196 were tested for the preparation of particles according to the invention.

100 mg of Polylactic acid (PLA) (R206; (Boehringer Ingelheim, Ingelheim am Rhein, Germany) and 40 mg lauric acid were each dissolved in 1 ml acetone. 100 µmol of EtOH soluble HAL salts (HCl, Etbs, Butbs, Hexbs) were dissolved in 1 ml EtOH. HAL Octbs and HAL DBS were dissolved in 1 ml acetone. 1 ml EtOH was added to the final drug-polymer solution. The PLA - , lauric acid - and HAL salt solution were mixed and completed to 10 ml with acetone.

Nanoparticles were prepared by nanoprecipitation as described in *Zeisser et al., 2006, above.* For this, the organic polymer-drug solution was added to an aqueous phase (20 ml of 0.4% PVAL solution in water) through a glass pipette and under continuous stirring. The solvents were left to evaporate at least 2 hours and until no acetone smell could be detected. The obtained nanosuspension was then filtered through a paper filter to discard large aggregates. For further purification, nanoparticles were centrifuged at 15'000g for 10 min, the supernatant was discarded and the particles were resuspended in 5 ml water. The procedure was repeated once. The nanoparticles were then lyophilised for 48 hrs.

### Loading and encapsulation efficiency

The nanoparticle loading was determined by dosing of the remaining HAL in the supernatant following nanoprecipitation. For this purpose, the HAL amount of 100 µl supernatant of the primary nanosuspension was determined by fluorescence by adding 300 µl of 1 mg/ml fluorescamine solution in acetone to 2.5 ml borate buffer pH 8.1 containing the sample solution. Mass loading was determined by calculating the ratio of encapsulated HAL-salt [mg] to the amount of nanoparticles. Molar loading was defined as the encapsulated amount of HAL-salt in µmol per 100 mg nanoparticles. Encapsulation efficiency (%) was determined by calculating the ratio of the encapsulated amount of HAL salt in mg versus the initial amount of HAL salt used for the production of nanoparticles. Table 2 below represents the size, polydispersity, mass loading, molar loading and encapsulation efficiencies for HAL salt laden PLA nanoparticles measured as described above.

**Table 2**

| Encapsulated salt | Size [nm] | Poly-dispersity | Mass loading [%] | Molar loading [µmol/100 mg nanos] | Encapsulation efficiency [%] |
|---|---|---|---|---|---|
| no salt | 309.2 | 0.03 | 0.0 | 0.0 | 0.0 |
| HAL HCl | 473.1 | 0.14 | 3.9 | 15.4 | 16.4 |
| HAL Etbs | 504.8 | 0.18 | 10.3 | 30.6 | 32.5 |
| HAL Butbs | 424.5 | 0.08 | 5.3 | 14.7 | 15.5 |
| HAL Hexbs | 444.9 | 0.14 | 15.7 | 40.0 | 45.9 |
| HAL Octbs | 406.5 | 0.16 | 28.1 | 66.8 | 92.1 |

Up to 25% loading can be achieved, depending on which HAL salt according to the invention was used, while the maximal loading using the hydrochloride salt was only 4% (loading for HAL-mesylate or salicylilate was <1%). Furthermore, with the HCl salt, encapsulation efficiency was poor (<17%) as compared to aryl sulfonate salts according to the invention (30 to 90 %).

### Particle characterization

The mean size of the nanoparticles was determined by photon correlation spectroscopy using a Zetasizer® 3000HS (Malvern instruments, Worcestershire, UK). The zeta potential was measured in 10⁻³ M NaCl using the electrophoretic mode with the Zetasizer® 3000HS. The measured polydispersity of all nanoparticle preparations is acceptable (<0.2). The unloaded nanoparticles have a size of about 300 nm, about 100 - 200 nm smaller than that of loaded nanoparticles.

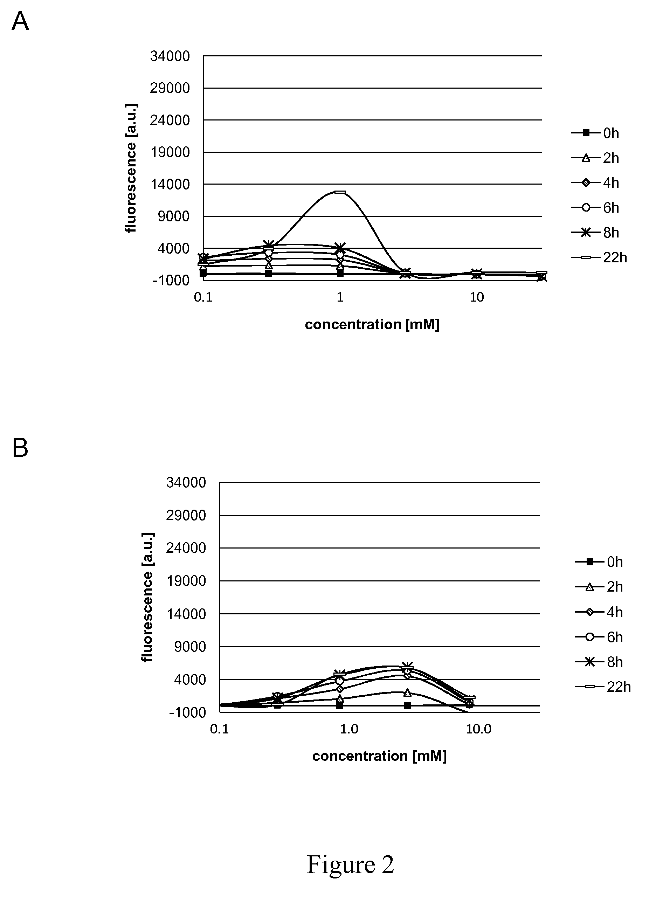

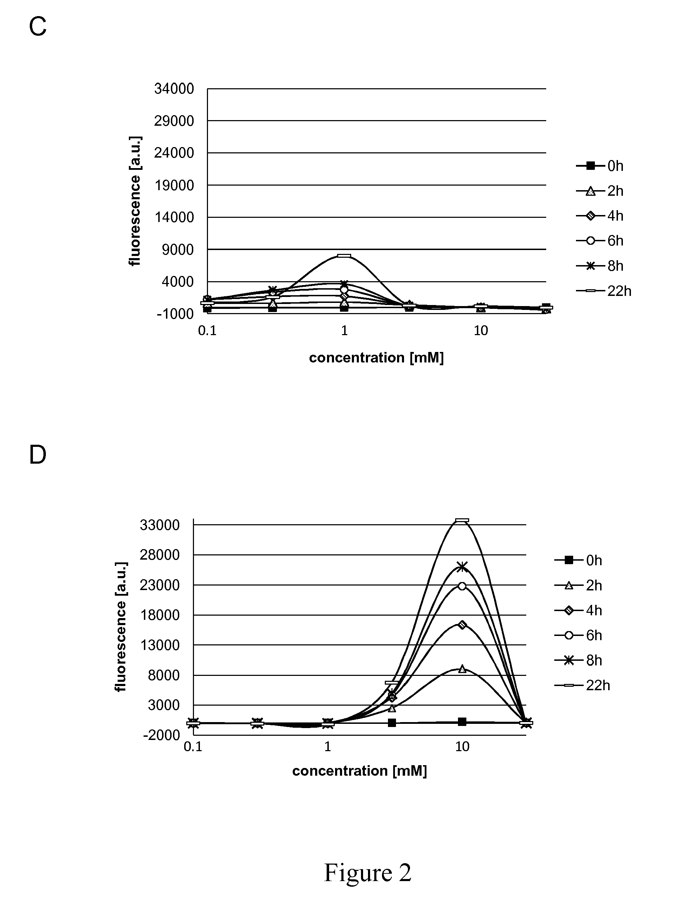

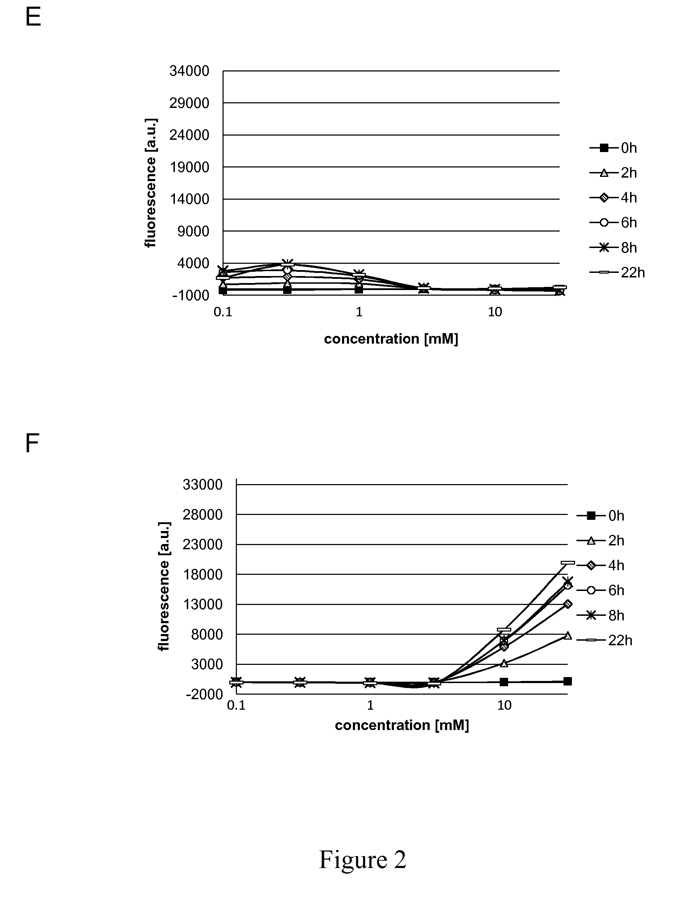

The morphology of loaded and unloaded nanoparticles was analysed by scanning electron microscopy (SEM) (JEOL JSM-7001FA, Tokyo, Japan). Samples were prepared by suspending approximately 1 mg nanoparticles in 1ml water. The suspensions were deposited on the sample holder and dried under vacuum for 24 hours. They were then coated with gold at 0.05 mbar for 90 seconds. The applied current was 15 mA and the sample distance to the cathode was 5 cm. Images were obtained with help of SMILE view software (Fig. 2). Morphology was similar for loaded and unloaded nanoparticles. Differential scanning calorimetry (DSC) was performed to examine the effect of encapsulated compounds on the polymer properties and to determine the crystalline state of the 5-ALA ester. 5 mg of lyophilised sample (HAL free salt or the HAL salt laden particles) were weighed into an aluminium pan (Perkin Elmer, Switzerland) and hermetically sealed. One heating cycle was run for free salts. For nanoparticles measurements were carried out over 2 heating cycles under nitrogen atmosphere. A sealed aluminium pan containing air was taken as reference. Samples were scanned with a heating rate of 5°C per minute. The system was cooled with liquid nitrogen. The DSC calorimeter (Seiko Instruments Inc., Japan) was calibrated with indium.

The calorigrams of free salts showed melting peaks, confirming that HAL-salts are small molecules that do not possess any Tg (Glass Transition Temperature). Unloaded nanoparticles feature a glass transition shoulder in their calorigram at 49°C, confirming the presence of a polymer.

Calorigrams of nanoparticles containing HAL Butbs, - Hexbs and Octbs show Tg shoulders at 48.8, 44.5 and 43.1°C, respectively. The absence of melting peaks shows that the salt compounds are homogeneously distributed throughout the polymer.

### Comparison with other HAL salts laden particles

When using AOT or DDS as counterions for the preparation of HAL laden nanoparticles strong aggregation was observed. Furthermore, resuspension of these nanoparticles was difficult.

Therefore, 5-ALA ester salts according to the invention are advantageous for the formation of particles according to the invention.

### Example 3: Porphyrin accumulation in PC-3 and T24 cells

The human prostate cancer cell line PC-3 (from laboratory cell stock), and human bladder cancer cell line T24 (Meddiscovery SA, Switzerland) were grown as monolayers. Cells were routinely maintained in DMEM-Glutamax-1 medium supplemented with 10% fetal calf serum (FCS), 100 µl/ml streptomycin and 100 I.U./ml penicillin. Cells were cultivated at 37°C in a regulated 95% air humidity and 5% CO₂ and seeded in new medium every 2-3 days. Then, cells were seeded into 96 well plates and allowed to attach overnight (until 90% confluence). The next day, they were rinsed with 200 µl HBSS and incubated with solutions containing either the free HAL salt or the corresponding HAL salt-laden nanoparticles prepared as described in Example 2. Immediately after incubation start, the porphyrin fluorescence emission was monitored over a wavelength span of 500 to 750 nm using a Saphire microplate reader (Saphire, Tecan, Männerdorf, Switzerland) using an excitation wavelength of 405 nm. Further measurements were performed two, four, six, eight, and 22 hours later, without removal of the incubation medium. After background subtraction, porphyrin accumulation was calculated according to the fluorescence intensity at 635 nm. No fluorescence was observed in cells incubated with control nanoparticles.

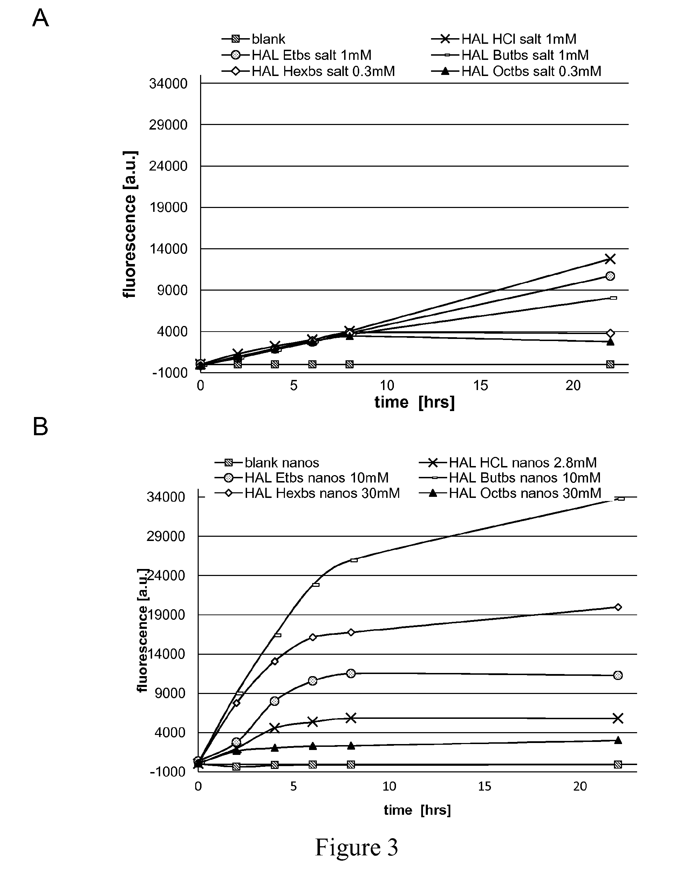

It is observed that none of the free salts of HAL induce more porphyrins than the hydrochloride salt (Fig. 3A, C & D). Furthermore, for the most lipophilic salts according to the invention (longer R¹ chains) the concentration for optimal porphyrin production is shifted slightly towards lower concentrations of HAL salt and fluorescence intensity is decreased (5 times less for Hexbs or Octbs) as compared to HAL HCl, indicating possible precipitation of the substrate for porphyrin synthesis at higher concentrations of HAL salt. This situation drastically changes when the same salts are encapsulated into PLA nanoparticles (Fig. 3B, C & D). For the most hydrophilic salts, HCl and Etbs, the porphyrin synthesis is almost as effective as for the respective free forms but the optimal concentration is shifted to higher concentrations of HAL salt. For the more lipophilic salts according to the invention, the optimal concentration is further shifted to the higher concentrations, indicating that these pharmaceutical forms provide a slow release of HAL salts from the polymeric matrix. Furthermore, a burst release can be excluded since assuming that half of the HAL salt is released in the medium would give a concentration of 5-15 mM of HAL salt which is potentially toxic to the cells.

Already for the encapsulated HAL Etbs salt, a notable porphyrin fluorescence intensity can be observed at 10 mM. At the respective optimal concentrations, encapsulated HAL Butbs induces three times more porphyrins than the free HAL hydrochloride, the current gold standard in 5-ALA-mediated phototherapy and fluorescence photodetection technologies. Already after 3 hours of incubation the same porphyrin concentration is obtained as induced by free HAL HCl after 22 hours.

The fluorescence kinetic profiles of porphyrin accumulation in human T24 cells at the optimal concentration for the free and nanoencapsulated forms, respectively (Fig. 6A and B) show that except for the HAL hydrochloride salt and the HAL Octbs salt, similar or higher porphyrin synthesis can be observed for nanoencapsulated forms. The observation that with HAL Octbs salt less porphyrin has been formed is attributed to the fact that the release from the nanoparticles is slower compared to their more hydrophilic salts. Therefore the concentration for optimal (highest) porphyrin formation is shifted towards higher concentrations of HAL salt. This was verified in human PC-3 cells, where the most important porphyrin production was obtained at a concentration of 47 mM in nanoencapsulated HAL Octbs salt, exceeding the fluorescence induction by the free salt by more than a factor of three, 26 hours after incubation. This further supports the observation of a slow but constant release of substrate for heme biosynthesis and indicates that the therapeutic index (ratio between the toxic dose and the therapeutic/diagnosis dose) is increased by at least a factor of 70 for a formulation according to the invention.

### Comparison with other HAL salts laden particles

HAL laden particles with AOT or DDS salt, although presenting relatively good encapsulation efficiencies (97.3% and 99.3% respectively), did not induce high amounts of PpIX in T24 cells (maximal fluorescence was 3200 arbitrary units [a.u.] for HAL AOT and 4450 a.u. for HAL DDS).

### Example 4: In vivo toxicity

Fertilized hen eggs were prepared as described previously in Vargas et al., 2007, Advanced Drug Delivery Reviews, 59, 1162-1176*.* On embryo development day 12, a hole in the egg shell was enlarged to a diameter of about 2-3 cm. Then, the corresponding the HAL salts according to the invention and or the corresponding nanoparticles comprising these salts were injected into one of the principle blood vessels in the chorioallantoic membrane and acute toxicity was evaluated with respect to the survival of the embryo 24 hours post injection.

Test for acute toxicity in chick embryos monitoring arrest of blood flow following injection have revealed that all free salts display higher and acute mortality (200 micromol/kg) than the hydrochloride analogue, whereas no mortality was observed with doses as high as 1000 micromol HAL/kg when the salts of the invention were encapsulated in PLA nanoparticles.

These results further support an increased safety for a formulation comprising particles according to the invention as compared to a formulation comprising free 5-ALA ester salts.

## Claims

1. A particle for systemic administration in a human or animal host, said particle comprising a biocompatible polymer and a salt of a 5-Aminolevulinic acid ester, wherein the salt is selected from an aryl sulfonate, an aryl phosphate salt and an aryl nitrate salt or a mixture thereof.

2. A particle according to claim 1, wherein the salt is selected from an aryl sulfonate of Formula (I), an aryl phosphate salt of Formula (II) and an aryl nitrate salt of Formula (III): 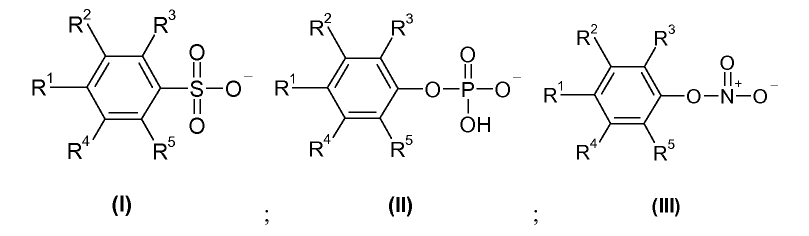 wherein R¹, R², R³, R⁴ and R⁵ are independently selected from H, optionally substituted C₁-C₂₀ alkyl, optionally substituted C₂-C₂₀ alkenyl, optionally substituted C₂-C₂₀ alkynyl, optionally substituted alkoxy, optionally substituted amino, halogen, hydroxyl and nitro, wherein at least one of R¹, R², R³, R⁴ and R⁵ is not H, or a mixture thereof.

3. A particle according to claims 1 or 2, wherein the salt is Formula (I), wherein R¹, R², R³, R⁴ and R⁵ are defined in any one of the preceding claims.

4. A particle according to any one of claims 2 to 3 wherein R¹ is an optionally substituted C₂-C₂₀ alkyl.

5. A particle according to any one of claims 2 or 4 wherein R¹ is selected from optionally substituted ethyl, optionally substituted butyl, optionally substituted hexyl, optionally substituted octyl, optionally substituted dodecyl.

6. A particle according to any one of claims 2 to 5 wherein R², R³, R⁴ and R⁵ are H.

7. A particle according to any one of claims 1 to 6 wherein the aryl sulfonate is selected from ethyl benzene sulfonate (Etbs), butyl benzene sulfonate (Butbs), hexyl benzene sulfonate (Hexbs), octyl benzene sulfonate (Octbs) and dodecyl benzene sulfonate (DBS).

8. A particle according to any one of claims 1 to 7 wherein the 5-Aminolevulinic acid ester is HAL.

9. A particle according to any one of claims 1 to 7, wherein the biocompatible polymer is PLA.

10. A particle according to any one of claims 1 to 9 for use as a medicament.

11. A pharmaceutical formulation comprising a particle according to any one of claims 1 to 9 and at least one pharmaceutically acceptable carrier.

12. A pharmaceutical formulation according to claim 11 further comprising a co-agent useful in the treatment of a cancer or a co-agent useful in the detection of a cancer cell.

13. A particle according to any one of claims 1 to 9 or a formulation according to claim 11 for the treatment or repression of a disorder or disease selected from tumors or other hyperproliferative conditions, skin disorders, skin cancer, actinic keratosis, infectious diseases, inflammatory diseases, Barrett's oesophagus and arterial restenosis.

14. A particle according to any one of claims 1 to 9 or a formulation according to claims 11 I or 12 for the treatment or repression of a cancer or for the detection of a cancer cell.

15. A process for the preparation of a particle for systemic administration in a human or animal host or a formulation thereof comprising a steps of:
(i) providing a solution comprising a 5-ALA ester salt selected from an aryl sulfonate, an aryl phosphate salt and an aryl nitrate salt and one or more biocompatible polymers and optionally a surfactant;
(ii) providing a solvent in which the said biocompatible polymer is not soluble; contacting the solution provided under step (i) solvent provided under step (ii);
(iii) membrane filtering the mixture obtained under step (iii);
(iv) collecting the particles filtered under step (iv).
